# EUROPÄISCHE PATENTSCHRIFT

(11) **EP 4 268 771 B1**
(45) Veröffentlichungstag und Bekanntmachung des Hinweises auf die Patenterteilung: **10.04.2024**
(21) Anmeldenummer: 22169675.0
(22) Anmeldetag: 25.04.2022
(51) Int. Cl.: A61F 2/30

(54) **AUGMENTATIONSVORRICHTUNG UND VERFAHREN ZUR ANPASSUNG EINER AUGMENTATIONSVORRICHTUNG**
AUGMENTATION DEVICE AND METHOD FOR ADJUSTING AN AUGMENTATION DEVICE
DISPOSITIF D'AUGMENTATION ET PROCÉDÉ D'AJUSTEMENT D'UN DISPOSITIF D'AUGMENTATION

(43) Veröffentlichungstag der Anmeldung: 01.11.2023
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- EP-B1- 2 130 518
- WO-A1-2021/013923
- US-A- 5 766 262
- US-A1- 2011 009 974
- US-A1- 2014 276 882
- US-A1- 2018 271 658

## Beschreibung

Die Erfindung ist in den Ansprüchen 1 und 9 definiert. Sie betrifft eine Augmentationsvorrichtung umfassend einen ringförmigen Konus, der einen Kanal umgibt, welcher sich von einem proximalen Konusende zu einem distalen Konusende des Konus durch den Konus erstreckt.

Die Erfindung betrifft weiterhin ein Verfahren zur Adaption einer Konusgröße einer derartigen Augmentationsvorrichtung.

Gegenstand der Erfindung ist insbesondere eine Augmentationsvorrichtung zum Einsatz bei Gelenkendoprothesenoperationen, insbesondere Revisionsgelenkendoprothesenoperationen. Die erfindungsgemäße Augmentationsvorrichtung ist dazu geeignet, debridiertes Knochengewebe zu verstärken oder gar teilweise zu ersetzen und so eine sichere und stabile Verankerung einer Gelenkendoprothese, insbesondere einer Revisionsendoprothese, in einem Knochenkanal zu ermöglichen. Zudem dient die erfindungsgemäße Augmentationsvorrichtung insbesondere einer gleichmäßigen Krafteinleitung in das umgebende Knochengewebe im Zuge einer Implantation einer Gelenkendoprothese, insbesondere einer Revisionsgelenkendoprothese

### Hintergrund der Erfindung

In der orthopädischen Chirurgie müssen leider in einem gewissen Umfang septische Revisionen von mit Mikroorganismen infizierten Gelenkendoprothesen vorgenommen werden. Dabei werden die infizierten Gelenkendoprothesen explantiert und das infizierte beziehungsweise nekrotische Gewebe abgetragen. Dieses Abtragen von infiziertem/nekrotischem Gewebe wird als Debridement bezeichnet. Dabei kann es im Bereich der entfernten Gelenkendoprothese zu einem substanziellen Verlust an Knochengewebe, insbesondere bei vorgeschädigter Knochengewebesubstanz, beispielsweise durch Osteoporose, kommen, was zu Problemen bei der Verankerung einer Revisionsgelenkendoprothese führen kann. Um das verbliebene Knochengewebe zu verstärken oder teilweise gar zu ersetzen und eine möglichst gleichmäßige Krafteinleitung in das verbliebene Knochengewebe beim Einsatz der Revisionsgelenkendoprothese zu gewährleisten, besteht eine mögliche Behandlungsoption in einem Einsatz einer Augmentationsvorrichtung in Form eines Metallkonus. Dazu wird das infizierte Knochengewebe abgetragen und die dadurch entstandene Kavität durch Einsetzten der Augmentationsvorrichtung aufgefüllt. Die konusförmige Ausgestaltung der Augmentationsvorrichtung dient dazu, einen Schaft einer Gelenkendoprothese, beziehungsweise einer Revisionsgelenkendoprothese, in einen zentral verlaufenden Kanal der Augmentationsvorrichtung aufzunehmen und dort, beispielsweise unter Einsatz von Knochenzement, zu verankern. Die Augmentationsvorrichtung selbst ist so gestaltet, dass sie in die durch das Debridement entstandene Kavität eingebracht werden kann. Eine derartige Augmentationsvorrichtung ist beispielsweise in der Patentschrift US 8,506,645 B2 beschrieben.

Üblicherweise weisen die im Markt erhältlichen Augmentationsvorrichtungen eine definierte, vorbestimmte Größe auf und können nicht an die jeweilige anatomische Gegebenheit des Patienten angepasst werden. Daher werden im Markt Augmentationsvorrichtungen unterschiedlicher Größe angeboten.

In der Patentschrift EP 3 319 558 B1 wird eine Augmentationsvorrichtung beschrieben, deren Größe, insbesondere deren Außendurchmesser, in bestimmten Grenzen variiert werden kann. Dazu weist die Augmentationsvorrichtung einen ringförmigen Konus auf, welcher zumindest ein axial verlaufendes Biegegelenk aufweist. Durch das zumindest eine Gelenk lässt sich der Konus durch einen von außen einwirkenden Druck zusammendrücken, was den Außendurchmesser der Augmentationsvorrichtung verringert.

Als nachteilig ist bei einer Verwendung von Gelenken, insbesondere Biegegelenken anzusehen, dass die Anpassung der Größe, insbesondere des Außendurchmessers, der Augmentationsvorrichtung mit einer deutlichen Verformung der Augmentationsvorrichtung einhergeht. Die Augmentationsvorrichtung weist durch die Anpassung der Größe einen "Knick" auf, welcher die Einpassung der Augmentationsvorrichtung in den Knochen des Patienten erschwert und sich nachteilig bei der Implantation der Gelenkendoprothese, beziehungsweise der Revisionsgelenkendoprothese, auswirken kann. Zudem ist eine Anpassung der Größe der Augmentationsvorrichtung nur in einem geringen Umfang möglich. Eine Anpassung der axialen Erstreckung der Augmentationsvorrichtung ist im Zuge einer laufenden Operation in einem Operationssaal nicht möglich.

In der Patentanmeldung US 2018/0271658 A1 wird eine Augmentationsvorrichtung aus einem Gewebe aus Titan beschrieben. Durch Abschneiden von Teilen des Gewebes kann die Augmentationsvorrichtung an die Anatomie eines Patienten angepasst werden.

Als nachteilig gestaltet sich an einer Augmentationsvorrichtung aus einem Metallgewebe, dass dieses nicht formstabil ist, was sowohl die Stabilität einer damit implantierten Prothese negativ beeinträchtigt. Zudem kann die bei der Implantation der Prothese benötigte Krafteinwirkung, welche auf die Augmentationsvorrichtung und das mit ihr verbundene Gewebe des Patienten einwirkt, nicht gleichmäßig übertragen werden. Insbesondere Letzteres kann zu Schädigungen am Knochengewebe des Patienten führen. Zudem kann das Abschneiden zu scharfkantigen Gewebesträngen führen, welche für den Operateur und den Patienten ein Verletzungsrisiko darstellen.

Ein Augment mit zwei längenanpassbaren Beinen wird in der Patentanmeldung US 2014/276882 A1 offenbart, deren technische Merkmale die Präambel des Anspruchs 1 bilden.

Wünschenswert ist daher eine Augmentationsvorrichtung, welche formstabil ist und einfach und schnell in ihrer Größe, insbesondere ihres Durchmessers und ihrer Länge, anpassbar ist. Die Anpassung der Größe soll ohne eine Verformung der Augmentationsvorrichtung möglich sein. Weiterhin soll die Anpassung der Größe mit Mitteln durchführbar sein, welche im Zuge einer orthopädischen Operation üblicherweise verwendet werden, wie beispielsweise Sägen.

### Aufgaben

Eine Aufgabe der vorliegenden Erfindung ist es, einen oder mehrere der sich aus dem Stand der Technik ergebenen Nachteile zumindest teilweise zu überwinden.

Insbesondere soll eine Augmentationsvorrichtung bereitgestellt werden, welche formstabil ist sowie einfach und schnell in ihrer Größe an die anatomischen Gegebenheiten eines Patienten angepasst werden kann. Weiterhin soll die Augmentationsvorrichtung bei einer Anpassung der Größe nicht verformt werden. Weiterhin soll die Augmentationsvorrichtung nach einer Anpassung der Größe kein Verletzungsrisiko darstellen.

Ein weiteres Ziel der Erfindung ist es ein Verfahren zur Verfügung zu stellen, mit dem eine Augmentationsvorrichtung in ihrer Größe angepasst werden kann und welches Beschränkungen herkömmlicher Implantationsverfahren vermeidet.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung gemäß Anspruch 1 ist eine Augmentationsvorrichtung umfassend einen ringförmigen Konus, der einen Kanal umgibt, welcher sich von einem proximalen Konusende zu einem distalen Konusende des Konus durch den Konus erstreckt, wobei der Konus zu mindestens 50 Volumenprozent, vorzugsweise zu mindestens 70 Volumenprozent, weiter bevorzugt zu mindestens 90 Volumenprozent, bezogen auf das Gesamtvolumen des Konus aus einem biokompatiblen Polymer besteht und durch mindestens drei, bevorzugt drei bis acht, weiter bevorzugt drei bis sechs, weiter bevorzugt drei bis fünf, in einer dem Kanal gegenüberliegenden Konusmantelfläche radial umlaufende Nuten in ringförmige Konussegmente unterteilt ist,
wobei die Nuten eine Nutentiefe von mindestens 1 mm und eine Nutenbreite von mindestens 1 mm aufweisen und Sägeführungen bilden, um eine Konusgröße des Konus durch Abtrennung eines oder mehrerer Konussegmente zu adaptieren.

Im Folgenden werden bevorzugte, teilweise durch die abhängigen Ansprüche abgebildete, Ausführungsformen eins bis acht beschrieben.

In einer Ausführungsform der Augmentationsvorrichtung ist die Konusmantelfläche zumindest abschnittweise aufgeraut oder porös. Diese Ausführungsform ist eine zweite Ausführungsform der Erfindung, welche vorzugsweise von der ersten Ausführungsform der Erfindung abhängt.

In einer Ausführungsform der Augmentationsvorrichtung ist die Konusmantelfläche zumindest zu 70 Flächenprozent, bevorzugt zumindest zu 80 Flächenprozent, weiter bevorzugt zumindest zu 90 Flächenprozent, bezogen auf die gesamte Konusmantelfläche aus Tantal, einer Tantallegierung, Titan, einer Titanlegierung oder aus Edelstahl gebildet. Diese Ausführungsform ist eine dritte Ausführungsform der Erfindung, welche vorzugsweise von der ersten oder der zweiten Ausführungsform der Erfindung abhängt.

In einer Ausführungsform der Augmentationsvorrichtung ist das biokompatible Polymer ein PMMA-Knochenzement. Diese Ausführungsform ist eine vierte Ausführungsform der Erfindung, welche vorzugsweise von einer der vorhergehenden Ausführungsformen der Erfindung abhängt.

In einer Ausführungsform der Augmentationsvorrichtung enthält der PMMA-Knochenzement mindestens ein Antibiotikum. Diese Ausführungsform ist eine fünfte Ausführungsform der Erfindung, welche vorzugweise von der vierten Ausführungsform der Erfindung abhängt.

In einer Ausführungsform der Augmentationsvorrichtung weist der Konus in einer dem Kanal zugewandten Konusinnenfläche radial umlaufende Innennuten auf, welche den Nuten gegenüberliegend, insbesondere radial gegenüberliegend, angeordnet sind. Diese Ausführungsform ist eine sechste Ausführungsform der Erfindung, welche vorzugweise von einer der vorhergehenden Ausführungsformen der Erfindung abhängt.

In einer Ausführungsform der Augmentationsvorrichtung weist die Konusmantelfläche zumindest zwei axial verlaufende Axialnuten mit einer Axialnutentiefe von mindestens 1 mm und einer Axialnutenbreite von mindestens 1 mm auf, welche jeweils zumindest mit einer der Nuten verbunden sind. Diese Ausführungsform ist eine siebte Ausführungsform der Erfindung, welche vorzugsweise von einer der vorhergehenden Ausführungsformen der Erfindung abhängt.

In einer Ausführungsform der Augmentationsvorrichtung sind die Konussegmente stufenartig voneinander abgesetzt. Diese Ausführungsform ist eine achte Ausführungsform der Erfindung, welche vorzugsweise von einer der vorhergehenden Ausführungsformen der Erfindung abhängt.

Eine neunte Ausführungsform der Erfindung ist ein Verfahren gemäß Anspruch 9 zur Adaption einer Konusgröße einer Augmentationsvorrichtung nach einer der vorhergehenden Ausführungsformen der Erfindung, umfassend einen Schritt eines Abtrennens eines oder mehrerer Konussegmente am proximalen Konusende, am distalen Konusende oder am proximalen und am distalen Konusende durch Sägen, Schneiden oder Abbrechen entlang der radial umlaufenden Nut oder Nuten.

Eine Ausführungsform des Verfahrens zur Adaption der Konusgröße einer Augmentationsvorrichtung nach der siebten Ausführungsform der Erfindung umfasst einen Schritt eines Abtrennens eines Konusteilstücks aus dem Konus durch Sägen, Schneiden oder Abbrechen entlang zweier der axial verlaufenden Axialnuten. Diese Ausführungsform ist eine zehnte Ausführungsform der Erfindung, welche vorzugsweise von der neunten Ausführungsform der Erfindung abhängt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Die Begriffe "proximal" und "distal" dienen lediglich der Bezeichnung der räumlich entgegengesetzten Enden der Augmentationsvorrichtung, des Konus, oder anderer Struktureinheiten der Augmentationsvorrichtung und des Augmentationssystems und erlauben keinen Rückschluss auf die Orientierung der in einen menschlichen Körper implantieren Augmentationsvorrichtung. "Distal zu ..." und "proximal zu..." oder ähnliche Formulierungen drücken entsprechend lediglich die räumliche Anordnung zweier Struktureinheiten der Augmentationsvorrichtung und des Augmentationssystems zueinander aus.

### Ausführliche Beschreibung

Ein erster Gegenstand der Erfindung betrifft eine Augmentationsvorrichtung umfassend einen ringförmigen Konus, der einen Kanal umgibt, welcher sich von einem proximalen Konusende zu einem distalen Konusende des Konus durch den Konus erstreckt, wobei der Konus zu mindestens 50 Volumenprozent, vorzugsweise zu mindestens 70 Volumenprozent, weiter bevorzugt zu mindestens 90 Volumenprozent, bezogen auf das Gesamtvolumen des Konus aus einem biokompatiblen Polymer besteht und durch mindestens drei, bevorzugt drei bis acht, weiter bevorzugt drei bis sechs, weiter bevorzugt drei bis fünf, in einer dem Kanal gegenüberliegenden Konusmantelfläche radial umlaufende Nuten in ringförmige Konussegmente unterteilt ist,
wobei die Nuten eine Nutentiefe von mindestens 1 mm und eine Nutenbreite von mindestens 1 mm aufweisen und Sägeführungen bilden, um eine Konusgröße des Konus durch Abtrennung eines oder mehrerer Konussegmente zu adaptieren.

Die Augmentationsvorrichtung weist einen ringförmigen Konus auf. Ein Konus ist ein kegelartiges, insbesondere kegelstumpfartig, geformtes Bauteil mit einem proximalen Konusende und einem dem proximalen Konusende axial gegenüberliegendem distalen Konusende, wobei sich ein Konusaußendurchmesser von einem Konusende zum anderen Konusende hin verringert. Vorzugsweise verjüngt sich der erfindungsgemäße Konus vom proximalen Konusende in Richtung des distalen Konusende.

Der Konus ist ringförmig, oder in anderen Worten rohrartig, ausgestaltet. Dadurch umschließt der Konus einen Kanal, welcher sich axial durch den Konus vom proximalen Konusende bis zum distalen Konusende erstreckt. Der Kanal wird durch eine dem Kanal zugewandten Konusinnenfläche ausgebildet. Der Begriff "ringförmig" umfasst in einer axialen Aufsicht auf den Konus Körper mit einem kreisförmigen, elliptischen, eckigen, beispielweise vier-, fünf- oder sechseckigen, und unregelmäßigen Umfang, wobei Körper mit elliptischem Umfang aufgrund einer besseren Implantierbarkeit in einen Patienten, insbesondere in einen Knochenkanal eines Patienten, bevorzugt sind.

Der Kanal dient der Aufnahme und Fixierung eines Schafts einer Gelenkendoprothese oder einer Revisionsgelenkendoprothese. Dafür kann der Schaft der entsprechenden Endoprothese in den Kanal, insbesondere vom proximalen Konusende aus, eingebracht werden. Der Kanal weist einen Kanaldurchmesser auf, welcher von einem Konusinnendurchmesser bestimmt ist. In einer Ausgestaltungsform kann der Konusinnendurchmesser über die gesamte axiale Erstreckung des Kanals beziehungsweise des Konus konstant ausgestaltet sein. In einer weiteren Ausgestaltungsform verringert sich der Konusinnendurchmesser gleichlaufend zum Konusaußendurchmesser vom proximalen Konusende bis zum distalen Konusende. In dieser Ausgestaltungsform weist der Konus eine über die gesamte axiale Erstreckung des Konus im Wesentlichen gleich starke Konuswanddicke auf.

Die Konuswanddicke kann beispielsweise in einem Bereich von 2 bis 30 mm, bevorzugt 2 bis 15 mm, weiter bevorzugt 2 bis 10 mm, liegen.

Die axiale Erstreckung des Konus kann beispielsweise in einem Bereich von 30 mm bis 50 mm, so dass die Augmentationsvorrichtung für eine möglichst breite Bandbreite von unterschiedlichen anatomischen Gegebenheiten von Patienten geeignet oder anpassbar ist.

Der Konus weist eine dem Kanal gegenüberliegende, und somit außenliegende, Konusmantelfläche auf. In der Konusmantelfläche verlaufen mindestens drei, vorzugsweise drei bis acht, weiter bevorzugt drei bis sechs, weiter bevorzugt drei bis fünf, radial umlaufende Nuten, welche den Konus in ringförmige Konussegmente unterteilen.

Nuten sind längliche Vertiefungen in der Konusmantelfläche. Die Nuten sind radial umlaufend und erstrecken sich vorzugsweise im Wesentlichen jeweils in einer Ebene, welche jeweils vorzugsweise im Wesentlichen senkrecht zu einer Längsachse der Augmentationsvorrichtung liegt. Die Nuten verlaufen somit im Wesentlichen "horizontal" zur Längsachse der Augmentationsvorrichtung.

Die Konuswanddicke ist im Bereich der Nuten reduziert, so dass der Konus durch die Nuten in ringförmige Konussegmente unterteilt ist. Beispielsweise unterteilen drei Nuten den Konus in vier Konussegmente und vier Nuten den Konus in fünf Konussegmente.

Die Nuten erleichtern das Abtrennen einzelner Konussegmente, so dass die Augmentationsvorrichtung eine adaptierbare Konusgröße, insbesondere einen adaptierbaren maximalen und/oder minimalen Konusaußendurchmesser und eine adaptierbare axiale Erstreckung des Konus, aufweist.

Die Nuten besitzen eine Nutentiefe von mindestens 1 mm und eine Nutenbreite von mindestens 1 mm, so dass die Nuten als Sägeführungen für Mittel zum Abtrennen einzelner Konussegmente, beispielsweise durch Sägen, Schneiden oder Abbrechen dienen. Geeignete Mittel zum Abtrennen sind beispielsweise Sägen, insbesondere Kreissägen, Zangen, und Scheren. Zum Abbrechen von Konussegmenten können beispielsweise Bruchkanten dienen. Um die strukturelle Stabilität des Konus nicht negativ zu beeinträchtigen, ist es bevorzugt, dass die Nutentiefe nicht mehr als einem Viertel (25%) der Konuswanddicke entspricht.

Um eine gute Sägeführung für Mittel zum Abtrennen einzelner Konussegmente darzustellen, ist es bevorzugt, dass die Nutenbreite nicht mehr als 5 mm, bevorzugt nicht mehr als 4 mm, weiter bevorzugt nicht mehr als 3 mm entspricht.

Die Nuten zeigen dabei dem Anwender geeignete Stellen zum Abtrennen einzelner Konussegmente an und erleichtern insbesondere ein kontrolliertes und sicheres Abtrennen einzelner Konussegmente entlang der entsprechenden Nut oder Nuten. Insbesondere beim Absägen und Abschneiden einzelner Konussegmente wird durch die Nuten ein Verletzungsrisiko für den Operateur, beispielsweise durch eine verringerte Gefahr eines Abrutschens einer Säge auf der Mantelfläche des Konus, vermindert. Die Nuten können darüber hinaus als Sollbruchstellen zum kontrollierten Abbrechen einzelner Konussegmente verwendet werden.

In einer Ausgestaltungsform erstrecken sich die Nuten jeweils um mindestens 90% des jeweiligen Konusaußendurchmessers. In einer weiteren Ausgestaltungsform erstrecken sich die Nuten jeweils um mindestens 95% des jeweiligen Konusaußendurchmessers. Vorzugsweise umlaufen die Nuten jeweils den gesamten jeweiligen Konusaußendurchmessers des Konus. Letzteres entspricht Nuten in Form eines geschlossenen "Rings".

Vorzugsweise sind die Nuten im Wesentlichen gleichmäßig über die Mantelfläche verteilt, so dass die einzelnen Konussegmente im Wesentlichen eine gleiche axiale Erstreckung aufweisen.

Um die Augmentationsvorrichtung möglichst gut an die anatomischen Gegebenheiten eines Patienten anpassen zu können, können die Konussegment unterschiedliche axiale Erstreckungen aufweisen. Beispielsweise sind die Nuten so angeordnet, dass die Konussegmente eine axiale Erstreckung im Bereich von 1 mm bis 10 mm, bevorzugt im Bereich von 2 mm bis 8 mm aufweisen.

Vorzugsweise ist der Konus massiv ausgestaltet, so dass die Konusinnenfläche und die Konusmantelfläche nicht durch den Konus hindurch, beispielsweise über eine Durchführung oder eine Leitung, miteinander fluidleitend verbunden sind. Ein massiver Konus verbessert die strukturelle Integrität der Augmentationsvorrichtung und erlaubt so eine verbesserte Krafteinleitung im Zuge einer Operation.

Der Konus besteht bezogen auf das Gesamtvolumen des Konus zumindest zu 50 Volumenprozent, bevorzugt zumindest zu 70 Volumenprozent, weiter bevorzugt zumindest zu 90 Volumenprozent aus einem biokompatiblen Polymer. Vorzugsweise ist die Konusinnenfläche vollständig aus dem biokompatiblen Polymer gebildet. In einer Ausgestaltungsform besteht der Konus im Wesentlichen vollständig aus einem biokompatiblen Polymer. Ein Vorteil eines biokompatiblen Polymers ist, dass dieses ein schnelles Anpassen der Konusgröße durch Abtrennen einzelner Konussegmente erlaubt und gleichzeitig dem Konus eine strukturelle Stabilität und damit Formstabilität gibt, welche eine gleichmäßige Kraftübertragung beim Implantieren der Augmentationsvorrichtung und einer entsprechenden Prothese ermöglicht. Zudem ergeben sich nach dem Abtrennen einzelner Konussegmente bei einem biokompatiblen Polymer lediglich Kanten, welche keine oder lediglich geringe Verletzungsgefahr für den Operateur darstellen. Weiter kann ein biokompatibles Polymer mit während einer chirurgischen Operation ohnehin zugänglichen Mitteln, wie beispielsweise Sägen, insbesondere Knochensägen, oder Scheren, bearbeitet werden, um die Konusgröße zu adaptieren.

Beispiele für biokompatible Polymere sind PEEK (Polyetherketon), PES (Polyethersulfon), Polyamide und Polyimide, wobei bei den Polyamiden Polyamid-12 bevorzugt ist.

Eine Ausführungsform der Augmentationsvorrichtung ist dadurch gekennzeichnet, dass das biokompatible Polymer ein PMMA-Knochenzement ist. Da üblicherweise die Befestigung einer Prothese im Kanal der Augmentationsvorrichtung mittels eines PMMA-Knochenzements erfolgt, bittet ein Konus ebenfalls aus einem PMMA-Knochenzement eine möglichst hohe Kompatibilität. Um die Kompatibilität mit dem zur Befestigung einer Prothese benutzten PMMA-Knochenzement zu erhöhen, ist die Konusinnenfläche bevorzugt im Wesentlichen vollständig aus einem PMMA-Knochenzement ausgebildet.

Der PMMA-Knochenzement kann mit weiteren Materialien beladen sein, wie beispielsweise Füllstoffen oder einem Röntgenopaker, wie beispielsweise Bariumsulft oder Zirkoniumoxid.

Eine Ausführungsform der Augmentationsvorrichtung ist dadurch gekennzeichnet, dass der PMMA-Knochenzement mindestens ein Antibiotikum enthält. Beispielsweise kann der PMMA-Knochenzement zwei, drei oder vier Antibiotika enthalten. Beispiele für Antibiotika sind Gentamicin, Tobramycin, Amikacin, Vancomycin, Teicoplanin, Ramoplanin, Daptomycin, Colistin und Meropenem, wobei Gentamycin und/oder Vancomycin bevorzugt ist.

Vorzugsweise enthält der PMMA-Knochenzement mindestens ein Antibiotikum, insbesondere mindestens Gentamycin oder Vancomycin, und ist im Wesentlichen frei von Röntgenopakern, da Röntgenopaker, insbesondere Zirkoniumoxid, aufgrund ihrer strukturellen Härte ein Abtrennen einzelner Konussegmente erschweren oder behindern könnten, beispielsweise durch ein Abstumpfen der Sägezähne einer Säge beim Abtrennen eines Konussegments entlang einer Nut.

In einer Ausführungsform der Augmentationsvorrichtung ist die Konusmantelfläche glatt ausgestaltet.

Eine Ausführungsform der Augmentationsvorrichtung ist dadurch gekennzeichnet, dass die Konusmantelfläche zumindest abschnittsweise, bevorzugt zumindest zu 80 Flächenprozent, weiter bevorzugt zumindest zu 90 Flächenprozent, jeweils bezogen auf die gesamte Konusmantelfläche, weiter bevorzugt im Wesentlichen vollständig, aufgeraut oder porös, vorzugsweise offen-porös, ist. Insbesondere die Konusmantelfläche zwischen und/oder neben den Nuten ist zumindest abschnittsweise, bevorzugt zumindest zu 80 Flächenprozent, weiter bevorzugt zumindest zu 90 Flächenprozent, jeweils bezogen auf die gesamte Konusmantelfläche, weiter bevorzugt im Wesentlichen vollständig, aufgeraut oder porös, vorzugsweise offen-porös. In einer Ausgestaltungsform ist die Mantelfläche mit Sackbohrlöchern versehen, um eine aufgeraute Mantelfläche bereitzustellen. Die Sackbohrlöcher haben dabei vorzugsweise einen Sackbohrlochdurchmesser m Bereich von 300 µm bis 500 µm. Eine aufgeraute oder poröse, vorzugsweise offen-poröse, Mantelfläche verbessert ein Ein- und/oder Anwachsen von Knochengewebe an und/oder in die Augmentationsvorrichtung und verbessert so die Stabilität der Augmentationsvorrichtung im implantierten Zustand. Eine derartige Mantelfläche bildet spongiöses Knochengewebe nach.

Die Mantelfläche kann aus dem biokompatiblen Polymer oder aus anderen biokompatiblen Materialien, wie beispielsweise biokompatiblen Metallen, bestehen oder diese umfassen.

Eine Ausführungsform der Augmentationsvorrichtung ist dadurch gekennzeichnet, dass die Konusmantelfläche zumindest zu 90 Flächenprozent, vorzugsweise zumindest zu 95 Flächenprozent, jeweils bezogen auf die gesamte Konusmantelfläche, weiter bevorzugt im Wesentlichen vollständig, aus Tantal, einer Tantallegierung, Titan, einer Titanlegierung oder aus Edelstahl besteht. Die vorgenannten Materialien verbessern ein Ein- und/oder Anwachsen von Knochengewebe an und/oder in die Augmentationsvorrichtung und verbessern so die Stabilität der Augmentationsvorrichtung im implantierten Zustand.

In einer Ausgestaltungsform wird die Mantelfläche aus Tantal, einer Tantallegierung, Titan, einer Titanlegierung oder aus Edelstahl durch ein Beschichtungsverfahren, wie beispielsweise Bedampfen, auf den Konus aus einem biokompatiblen Polymer bereitgestellt. Dabei kann die Schicht aus Tantal, einer Tantallegierung, Titan, einer Titanlegierung oder aus Edelstahl eine Schichtdicke in einem Bereich von 10 µm bis 1000 µm.

In einer weiteren Ausgestaltungsform wird die Mantelfläche aus Tantal, einer Tantallegierung, Titan, einer Titanlegierung oder aus Edelstahl als Teilkonus bereitgestellt, welcher mit einem weiteren Teilkonus aus dem biokompatiblen Polymer, beispielsweise durch Verklebung, Verklemmung oder Verschweißung, verbunden wird, um die Augmentationsvorrichtung bereitzustellen. Dabei kann die Schicht aus Tantal, einer Tantallegierung, Titan, einer Titanlegierung oder aus Edelstahl eine Schichtdicke in einem Bereich von 1 mm bis 7 mm.

Vorzugsweise besteht die Augmentationsvorrichtung aus einem Konus, welcher zumindest zu 50 Volumenprozent, bevorzugt zumindest zu 70 Volumenprozent, weiter bevorzugt zumindest zu 90 Volumenprozent aus einem biokompatiblen Polymer besteht, wobei der Knochenzement vorzugsweise mindestens ein Antibiotikum enthält, und wobei die restlichen Volumenprozent aus der aus Tantal, einer Tantallegierung, Titan, einer Titanlegierung oder aus Edelstahl bestehenden Konusmantelfläche bestehen.

In einer Ausführungsform ist die Konusinnenfläche des Konus glatt ausgeformt.

Eine Ausführungsform der Augmentationsvorrichtung ist dadurch gekennzeichnet, dass der Konus in der dem Kanal zugewandten Konusinnenfläche radial umlaufende Innennuten aufweist, welche den Nuten gegenüberliegend angeordnet sind. Vorzugsweise ist jeder Nut eine gegenüberliegende, insbesondere radial gegenüberliegende, Innennut angeordnet. Die Nut und die ihr gegenüberliegende Innennut verringern die Manteldicke des Konus, so dass einzelne Konussegmente vereinfacht von der Augmentationsvorrichtung, insbesondere durch Sägen, Schneiden oder Abbrechen, abtrennbar sind.

Innennuten sind, ebenso wie die Nuten, längliche Vertiefungen. Während sich die Nuten in der Konusmantelfläche erstrecken, erstrecken sich die Innennuten hingegen in der Konusinnenfläche. Die Innennuten sind radial umlaufend und erstrecken sich vorzugsweise im Wesentlichen jeweils in einer Ebene, welche jeweils vorzugsweise im Wesentlichen senkrecht zu einer Längsachse der Augmentationsvorrichtung liegt. Die Innennuten verlaufen somit im Wesentlichen "horizontal" zur Längsachse der Augmentationsvorrichtung. Die Nuten und die ihnen jeweils gegenüberliegend angeordneten Innennuten verlaufen vorzugsweise im Wesentlichen in einer gemeinsamen Ebene, welche im Wesentlichen senkrecht zur Längsachse der Augmentationsvorrichtung liegt.

Vorzugsweise weisen die Innennuten eine Innennutentiefe in einem Bereich von 1 mm bis zu 25 % der Wanddicke des Konus und eine Innennutbreite in einem Bereich von 1 mm bis maximal 5 mm, bevorzugt nicht mehr als 4 mm, weiter bevorzugt nicht mehr als 3 mm auf. Dies erleichtert ein Abtrennen einzelner Konussegmente zur Adaption der Konusgröße und erhält eine ausreichende strukturelle Integrität der Augmentationsvorrichtung, so dass beim Implantieren einer Prothese über die Augmentationsvorrichtung eine gleichmäßige Krafteintragung stattfinden kann.

Eine Ausführungsform der Augmentationsvorrichtung ist dadurch gekennzeichnet, dass die Konusmantelfläche zumindest zwei axial verlaufende Axialnuten mit einer Axialnutentiefe von mindestens 1 mm und einer Axialnutenbreite von mindestens 1 mm aufweist, welche jeweils zumindest mit einer der Nuten verbunden ist. Die Axialnuten erstrecken sich insbesondere in Richtung der Längsachse der Augmentationsvorrichtung.

Durch die Axialnuten ist ein Abtrennen eines axial verlaufendes Konusteilstücks zwischen zweier der Axialnuten vereinfacht. Das Abtrennen eines Konusteilstücks ermöglicht eine verbesserte Anpassung der Augmentationsvorrichtung an die anatomischen Gegebenheiten eines Patienten. Vorzugsweise weist der Konus zumindest vier Axialnuten auf, so dass durch ein Abtrennen entlang jeweils zweiter der Axialnuten zumindest zwei disjunkte Konusteilstücke vereinfacht abtrennbar sind. Vorzugsweise weisen jeweils zwei der Axialnuten eine radialen Abstand zueinander in einem Bereich von 2 mm bis 5 mm auf, so dass entsprechende Konusteilstücke mit einer Breite in diesem Bereich abtrennbar sind.

Die Axialnuten besitzen eine Nutentiefe von mindestens 1 mm und eine Axialnutenbreite von mindestens 1 mm, so dass die Axialnuten als Sägeführungen für Mittel zum Abtrennen eines Konusteilstücks, beispielsweise durch Sägen, Schneiden oder Abbrechen dienen. Geeignete Mittel zum Abtrennen sind beispielsweise Sägen, insbesondere Kreissägen, Zangen, und Scheren.

Um die strukturelle Stabilität des Konus nicht negativ zu beeinträchtigen, ist es bevorzugt, dass die Axialnutentiefe nicht mehr als einem Viertel (25%) der Konuswanddicke entspricht.

Um eine gute Führung für Mittel zum Abtrennen eines Konusteilstücks darzustellen, ist es bevorzugt, dass die Axialnutenbreite nicht mehr als 5 mm, bevorzugt nicht mehr als 4 mm, weiter bevorzugt nicht mehr als 3 mm entspricht.

Die Axialnuten zeigen dabei dem Anwender geeignete Stellen zum Abtrennen eines Konusteilstücks an und erleichtern insbesondere ein kontrolliertes und sicheres Abtrennen eines Konusteilstücks entlang der entsprechenden Axialnuten. Insbesondere beim Absägen und Abschneiden eines Konusteilstücks wird durch die Axialnuten eine Verletzungsrisiko für den Operateur, beispielsweise durch Verringern einer Abrutschgefahr einer Säge auf der Konusmantelfläche, vermindert. Die Axialnuten können auch als Sollbruchstellen zum kontrollierten Abbrechen eines Konusteilstücks verwendet werden.

Um ein Konusteilstück von dem Konus abzutrennen, kann beispielsweise entlang zweier Axialnuten gesägt werden. Vorzugsweise erstrecken sich die Axialnuten vom proximalen Konusende in Richtung des distalen Konusendes, so dass zumindest im Bereich des proximalen Konusendes ein vereinfachtes Abtrennen eines Konusteilstücks ermöglicht ist.

Die Axialnuten sind zumindest mit einer der Nuten verbunden. Beispielsweise können die Axialnuten in einer Nut münden oder sich axial über eine Nut hinaus erstrecken. Da die Axialnuten mit mindestens einer Nut verbunden sind, ist ein vereinfachtes Abtrennen eines Konusteilstücks entlang zweier der Axialnuten und entlang der mindestens einen Nut ermöglicht.

In einer Ausgestaltungsform erstrecken sich die Axialnuten über eine der Nuten hinaus und sind zumindest an ihren jeweiligen distalen Axialnutenenden über eine Verbindungsnut miteinander verbunden. Beispielsweise sind die Axialnuten mit einer oder zwei Verbindungsnuten, welche sich zwischen mindestens zwei der Nuten zwischen den Axialnuten erstrecken, miteinander verbunden. Dies ermöglicht ein vereinfachtes Abtrennen eines Konusteilstücks, welches eine axiale Erstreckung aufweist, welche nicht zwangsweise einem vielfachen der axialen Erstreckung eines Konussegments entspricht.

In einer Ausgestaltungform verlaufen die mindestens zwei Axialnuten über die gesamte axiale Erstreckung des Konus, so dass ein Abtrennen eines Konusteilstücks mit einer axialen Erstreckung, welcher der axialen Erstreckung des Konus entspricht, vereinfacht von dem Konus ermöglicht ist. Der resultierende Konus liegt nach dem Abtrennen des Konusteilstücks in Form eines offenen Rings vor.

In einer Ausführungsform der Augmentationsvorrichtung ist die Mantelfläche stufenlos ausgestaltet, so dass sich der Konusaußendurchmesser von einem Konusende zum anderen Konusende, vorzugsweise vom proximalen Konusende zum distalen Konusende, gleichlaufend verringert.

Eine Ausführungsform der Augmentationsvorrichtung ist dadurch gekennzeichnet, dass die Konussegmente stufenartig voneinander abgesetzt sind. Dadurch verringert sich der Konusaußendurchmesser stufenartig von einem der Konussegmente zum dessen benachbarten Konussegment. Vorzugsweise verlaufen die Nuten zwischen benachbarten stufenartiger Konussegmente derart, dass bei einem Abtrennen entlang der entsprechenden Nut ein im Wesentlichen vollständiges Trennen der beiden Konussegmente voneinander ermöglicht ist.

Ein weiterer Gegenstand der Erfindung betrifft ein Verfahren zur Adaption einer Konusgröße einer Augmentationsvorrichtung nach einer der vorhergehenden Ausführungsformen umfassend einen Schritt eines Abtrennens eines oder mehrerer Konussegmente am proximalen Konusende, am distalen Konusende oder am proximalen Konusende und am distalen Konusende durch Sägen, Schneiden oder Abbrechen entlang der radial umlaufenden Nut oder Nuten.

Um die Konusgröße in Form eines maximalen Konusaußendurchmesser zu verringern, werden ein oder mehrere Konussegmente am Konusende mit dem größeren Konusaußendurchmesser, vorzugsweise ist dies das proximale Konusende, abgetrennt.

Um die Konusgröße in Form eines minimalen Konusaußendurchmesser zu vergrößern, werden ein oder mehrere Konussegmente am Konusende mit dem geringeren Konusaußendurchmesser, vorzugsweise ist dies das distale Konusende, abgetrennt.

Um sowohl den maximalen Konusaußendurchmesser als auch den minimalen Konusaußendurchmesser anzupassen, werden sowohl am proximalen als auch am distalen Konusende mindestens ein Konussegment abgetrennt.

Jede Abtrennung eines Konussegments verringert die axiale Erstreckung der Augmentationsvorrichtung.

Um die Augmentationsvorrichtung noch besser an die anatomischen Gegebenheiten eines Patienten anzupassen, ist eine Ausführungsform des Verfahrens dadurch gekennzeichnet, dass das Verfahren zusätzlich einen Schritt eines Abtrennens eines Konusteilstückes aus dem Konus durch Sägen, Schneiden oder Abbrechen entlang zweier der axial verlaufenden Axialnuten umfasst. Für diese Ausführungsform wird eine Augmentationsvorrichtung mit mindestens zwei Axialnuten eingesetzt. In einer Ausgestaltungsform des Verfahrens werden aus dem Konus zwei Konusteilstücke durch Sägen, Schneiden oder Abbrechen entlang zweier Paaren an Axialnuten abgetrennt. Vorzugsweise sind die Konusteilstücke an gegenüberliegenden Positionen, vorzugsweise beide am proximalen Konusende, angeordnet. Das Abtrennen des Konusteilstückes kann vor oder nach dem Abtrennen eines oder mehrerer Konussegmente erfolgen.

Der Konus der Augmentationsvorrichtung besteht vorzugsweise zu mindestens 50 Volumenprozent, vorzugsweise zu mindestens 70 Volumenprozent, weiter bevorzugt zu mindestens 90 Volumenprozent, bezogen auf das Gesamtvolumen des Konus aus einem biokompatiblen Polymer in Form eines PMMA-Knochenzement (Polymethylmethacrylat-Knochenzement). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin.

Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

Die für die Augmentationsvorrichtung offenbarten Merkmale sind auch für das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: eine schematische Seitenansicht einer Augmentationsvorrichtung,
- Fig. 2: eine weitere schematische Seitenansicht der Augmentationsvorrichtung aus Figur 1,
- Fig. 3: einen schematischen Längsschnitt der Augmentationsvorrichtung aus den Figuren 1 bis 3,
- Fig. 4: einen vergrößerten Ausschnitt des schematischen Längsschnitts aus Figur 4,
- Fig. 5: eine schematische Seitenansicht der Augmentationsvorrichtung aus den Figuren 1 und 2 mit adaptierter Konusgröße,
- Fig. 6: einen schematischen Längsschnitt einer weiteren Augmentationsvorrichtung, und
- Fig. 7: eine schematische Seitenansicht einer weiteren Augmentationsvorrichtung mit stufenartig abgesetzten Konussegmenten.

### Beschreibung der Figuren

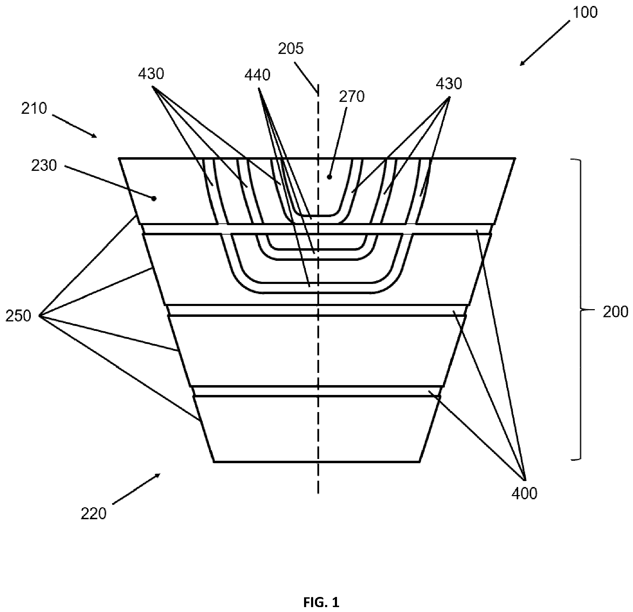

**Figur 1** zeigt eine schematische Seitenansicht einer beispielhaften Ausführung einer Augmentationsvorrichtung 100. Die Augmentationsvorrichtung 100 ist einteilig ausgeformt und besteht aus einem PMMA-Knochenzement beladen mit einem Antibiotikum. Die Augmentationsvorrichtung 100 umfasst einen massiven ringförmigen Konus 200 mit einem proximalen Konusende 210 und einem distalen Konusende 220, wobei sich ein Konusaußendurchmesser, gebildet durch eine Mantelfläche 230 des Konus, vom proximalen Konusende 210 in Richtung des distalen Konusende 220 abnimmt. In der gezeigten Ausführungsform nimmt der Konusaußendurchmesser vom proximalen Konusende 210 zum distalen Konusende 220 im Wesentlichen linear ab.

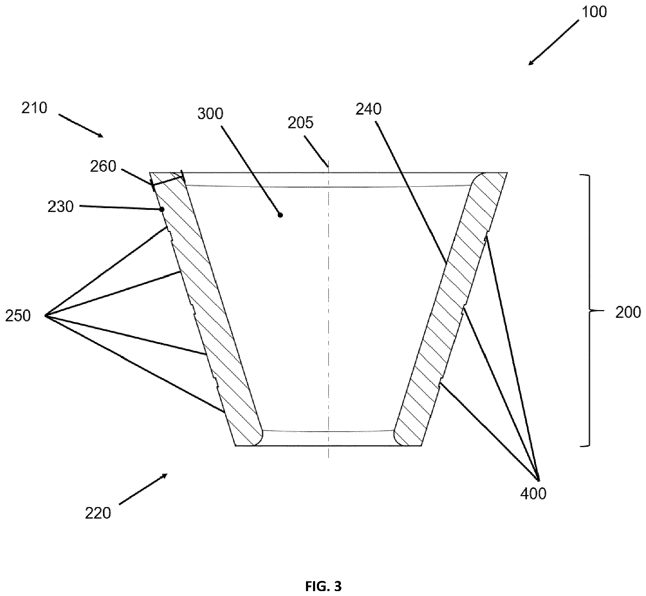

Durch den Konus 200 erstreckt sich vom proximalen Konusende 210 bis zum distalen Konusende 220 ein Kanal (siehe Figur 3).

In der Mantelfläche 230 des Konus 200 verlaufen jeweils radial vollständig umlaufend drei Nuten 400, welche den Konus in vier ringförmige Konussegmente 250 unterteilen. Dabei sind die Nuten 400 derart angeordnet, dass die Konussegmente 250 eine im Wesentlichen gleich große axiale Erstreckung aufweisen. Die Nuten 400 dienen als Sägeführungen, so dass ein oder mehrere Konussegmente 250 von der Augmentationsvorrichtung 100 mit geeigneten Mitteln, beispielsweise mit einer Säge, vereinfacht und sicher abtrennbar sind. Mit jedem Abtrennen eines einzelnen Konussegments 250 wird eine Konusgröße der Augmentationsvorrichtung 100 adaptiert. Beispielsweise wird durch ein Abtrennen eines Konussegments 250 am proximalen Konusende 210 sowohl die axiale Erstreckung des Konus 200 um im Wesentlichen 25% reduziert als auch ein maximaler Konusaußendurchmesser des Konus 200 verringert.

Die Nuten 400 erstrecken sich jeweils in einer Ebene, welche sich senkrecht zu einer Längsachse 205 des Konus 200 erstreckt.

In der Mantelfläche 230 verlaufen weiterhin zwölf Axialnuten 430 (lediglich sechs sind in Figur 1 zu sehen, die restlichen sechs sind auf einer der gezeigten Ansicht gegenüberliegenden Seite des Konus 200 angeordnet). Die Axialnuten 430 erstrecken sich über das Konusegment 250 am proximalen Konusende 210 und teilweise auch über das dazu distal benachbarte Konussegment 250. Alle Axialnuten 440 sind zumindest mit der Nut 400 am proximalen Konusende 210 verbunden, wobei vier der Axialnuten 440 in dieser Nut 400 münden, während die restlichen Axialnuten 400 sich bis weiter in Richtung des distalen Konusende 220 über die Nut 400 am proximalen Konusende 210 hinaus erstrecken.

Jeweils zwei der Axialnuten 430 sind über eine Verbindungsnut 440 miteinander verbunden und bilden ein Axialnutenpaar. Die Axialnuten 440 bilden mit der Nut 400 am proximalen Konusende 210 und/oder mit den Verbindungsnuten 440 Konusteilstücke 270 (lediglich exemplarisch mit einem Bezugszeichen versehen) aus, welche vereinfacht und sicher aus dem Konus 200, beispielsweise durch Sägen, abtrennbar sind und eine verbesserte Anpassung an die anatomischen Gegebenheiten eines Patienten erlauben.

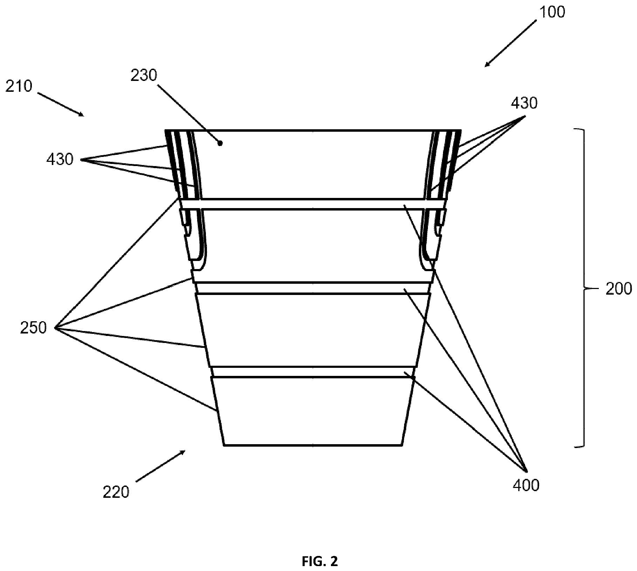

**Figur 2** zeigt die Augmentationsvorrichtung 100 aus Figur 1 in einer um 90° um die Längsachse gedrehten Seitenansicht. In Figur 2 ist ersichtlich, dass die Axialnuten 430 symmetrisch angeordnet sind, so dass Konusteilstücke 270 (vgl. Figur 1) auf zwei Seiten am proximalen Konusende 210 aus dem Konus 200 abtrennbar sind.

**Figur 3** zeigt die Augmentationsvorrichtung 100 aus den Figuren 1 und 2 in einem schematischen Längsschnitt. Figur 3 ist zu entnehmen, dass der Kanal 300 durch eine Konusinnenfläche 240 gebildet wird und sich vom proximalen Konusende 210 durch den Konus 200 bis zum distalen Konusende 220 erstreckt. Der Konus 200 weist eine Konuswanddicke 260 auf, welche, bis auf leichte Varianzen am proximalen Konusende 210 und am distalen Konusende 220, über die gesamte axiale Erstreckung konstant ist. Die Konuswanddicke 260 der gezeigten Ausführungsform der Augmentationsvorrichtung 100 beträgt 10 mm. Weiterhin ist zu erkennen, dass der Konus 200 massiv ausgestaltet ist und es somit keine fluidleitende Verbindung zwischen der Konusinnenfläche 240 und der Konusmantelfläche 230 durch den Konus 200 hindurch gibt.

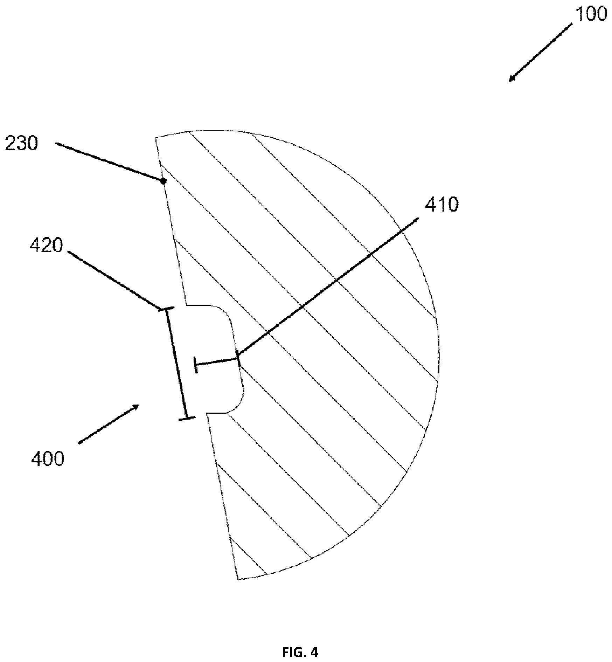

**Figur 4** zeigt einen vergrößerten Ausschnitt des schematischen Längsschnitts der Figur 3. Figur 4 zeigt insbesondere einen vergrößerten Längsschnitt durch eine der Nuten 400 der Augmentationsvorrichtung 100. Die Nut 400 weist eine senkrecht zur Mantelfläche 230 verlaufende Nutentiefe 410 und eine parallel zur Mantelfläche 230 verlaufende Nutenbreite 420 auf. In der gezeigten Ausführungsform der Augmentationsvorrichtung 100 besitzt die Nut 40 eine Nutentiefe 410 von 1 mm und eine Nutenbreite 420 von 2,5 mm.

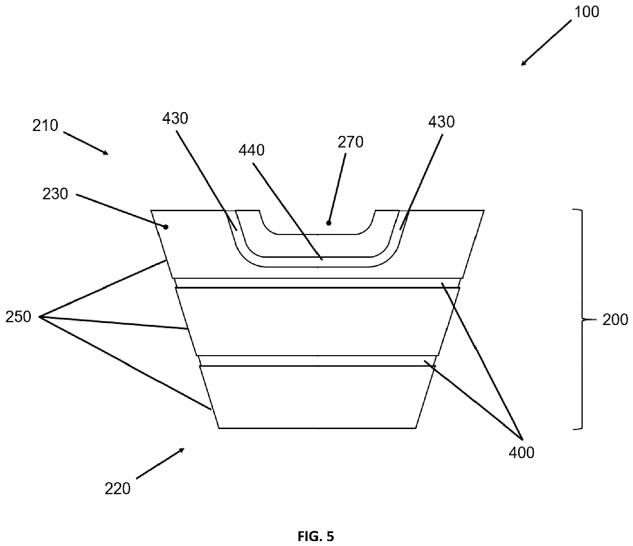

**Figur 5** zeigt die Augmentationsvorrichtung 100 aus den Figuren 1 bis 4 in einer schematischen Seitenansicht mit angepasster Konusgröße. Im Vergleich zu den vorhergehenden Figuren 1 bis 4 wurde das Konussegment 250 mit dem größten Außendurchmesser am proximalen Konusende 210 entfernt, so dass die Augmentationsvorrichtung 100 in Figur 3 einen verringerten maximalen Konusaußendurchmesser aufweist. Zudem wurde auf beiden Seiten des Konus 200 ein Konusteilstück 270 jeweils entlang des auf beiden Konusseiten verlaufenden mittleren Paar der Axialnuten 430 und deren Verbindungsnut 440 abgetrennt (vgl. Figur 1 und 2).

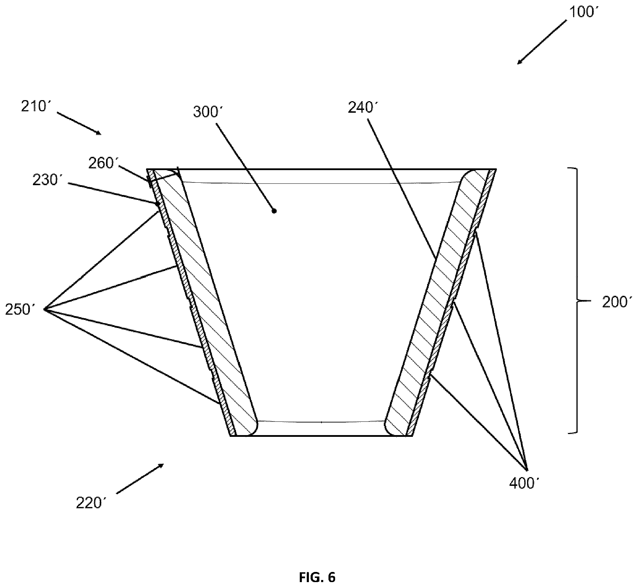

**Figur 6** zeigt einen schematischen Längsschnitt einer weiteren beispielhaften Augmentationsvorrichtung 100'. Die Ausführungsform der Augmentationsvorrichtung 100' stimmt weitgehend mit der vorstehend beschriebenen und in den Figuren 1 bis 5 dargestellten Ausführungsform überein, so dass zur Vermeidung von Wiederholungen auf die vorstehende Beschreibung verwiesen wird. Abwandlungen einer gegenüber der in den Figuren 1 bis 5 gezeigten Ausführungsform weisen dasselbe Bezugszeichen mit einem Apostroph auf.

Die Augmentationsvorrichtung 100' unterscheidet sich von der Augmentationsvorrichtung 100 aus den Figuren 1 bis 5 durch eine aus Titan gebildete poröse Mantelfläche 230'. In der gezeigten Ausführungsform der Augmentationsvorrichtung 100' ist die Mantelfläche 230' vollständig aus Titan gebildet.

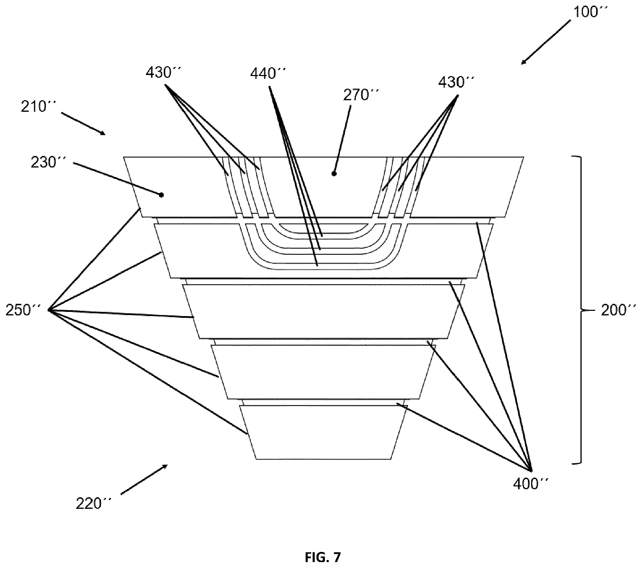

**Figur 7** zeigt eine schematische Seitenansicht einer weiteren beispielhaften Augmentationsvorrichtung 100". Die Ausführungsform der Augmentationsvorrichtung 100" stimmt weitgehend mit der vorstehend beschriebenen und in den Figuren 1 bis 5 und Figur 6 dargestellten Ausführungsformen überein, so dass zur Vermeidung von Wiederholungen auf die vorstehenden Beschreibungen verwiesen wird. Abwandlungen einer gegenüber der in den Figuren 1 bis 5 und Figur 6 gezeigten Ausführungsform weisen dasselbe Bezugszeichen mit zwei Apostrophen auf.

Die Augmentationsvorrichtung 100" unterscheidet sich von den Augmentationsvorrichtungen 100 und 100' aus den Figuren 1 bis 5 und Figur 6 durch stufenartig voneinander abgestufte Konussegmente 250". Durch die stufenartigen Konussegmente 250" verringert sich der Konusaußendurchmesser der Augmentationsvorrichtung 100" nicht im Wesentlichen linear vom proximalen Konusende 210" zum distalen Konusende 220", sondern "sprungartig" zwischen den Konussegmenten 250".

### Bezugszeichen

- 100, 100', 100": Augmentationsvorrichtung
- 200, 200', 200": Konus
- 205: Längsachse des Konus
- 210, 210', 210": proximales Konusende
- 220, 220', 220": distales Konusende
- 230, 230', 230": Konusmantelfläche
- 240, 240': Konusinnenfläche
- 250, 250', 250": Konussegment
- 260,260': Konuswanddicke
- 270, 270": Konusteilstück
- 300, 300': Kanal
- 400, 400', 400": Nut
- 410: Nutentiefe
- 420: Nutenbreite
- 430, 430": Axialnut
- 440, 440": Verbindungsnut

## Patentansprüche

1. Augmentationsvorrichtung (100, 100', 100") umfassend
einen ringförmigen Konus (200, 200', 200"), der einen Kanal (300, 300') umgibt, welcher sich von einem proximalen Konusende (210, 210', 210") zu einem distalen Konusende (220, 220', 220") des Konus (200, 200', 200") durch den Konus (200, 200', 200") erstreckt,
und welcher zu mindestens 50 Volumenprozent bezogen auf das Gesamtvolumen des Konus (200, 200', 200") aus einem biokompatiblen Polymer besteht,
**dadurch gekennzeichnet, dass**
der Konus (200, 200', 200") durch mindestens drei in einer dem Kanal (300, 300') gegenüberliegenden Konusmantelfläche (230, 230', 230") radial umlaufende Nuten (400, 400', 400") in ringförmige Konussegmente (250, 250', 250") unterteilt ist,
wobei die Nuten (400, 400', 400") eine Nutentiefe (410) von mindestens 1 mm und eine Nutenbreite (420) von mindestens 1 mm aufweisen und Sägeführungen bilden, um eine Konusgröße des Konus (200, 200', 200") durch Abtrennung eines oder mehrerer Konussegmente (250, 250', 250") zu adaptieren.

2. Augmentationsvorrichtung (100, 100', 100") nach Anspruch 1, **wobei** die Konusmantelfläche (230, 230', 230") zumindest abschnittsweise aufgeraut oder porös ist

3. Augmentationsvorrichtung (100') nach Anspruch 1 oder 2, **wobei** die Konusmantelfläche (230') zumindest zu 70 Flächenprozent bezogen auf die gesamte Konusmantelfläche (230') aus Tantal, einer Tantallegierung, Titan, einer Titanlegierung oder aus Edelstahl gebildet ist.

4. Augmentationsvorrichtung (100, 100', 100") nach einem der vorhergehenden Ansprüche, **wobei** das biokompatible Polymer ein PMMA-Knochenzement ist.

5. Augmentationsvorrichtung (100, 100', 100") nach Anspruch 4, **wobei** der PMMA-Knochenzement mindestens ein Antibiotikum enthält.

6. Augmentationsvorrichtung (100, 100', 100") nach einem der vorhergehenden Ansprüche, **wobei** der Konus (200, 200', 200") in einer dem Kanal (300, 300') zugewandten Konusinnenfläche (240, 240') radial umlaufende Innennuten aufweist, welche den Nuten (400, 400', 400") gegenüberliegend angeordnet sind.

7. Augmentationsvorrichtung (100, 100', 100") nach einem der vorhergehenden Ansprüche, **wobei** die Konusmantelfläche (230, 230', 230") zumindest zwei axial verlaufende Axialnuten (430, 430") mit einer Axialnutentiefe von mindestens 1 mm und einer Axialnutenbreite von mindestens 1 mm aufweist, welche jeweils zumindest mit einer der Nuten (400, 400', 400") verbunden sind.

8. Augmentationsvorrichtung (100) nach einem der vorhergehenden Ansprüche, **wobei** die Konussegmente (250") stufenartig voneinander abgesetzt sind.

9. Verfahren zur Adaption einer Konusgröße einer Augmentationsvorrichtung (100, 100', 100") nach einem der Ansprüche 1 bis 8 umfassend einen Schritt eines Abtrennens eines oder mehrerer Konussegmente (250, 250', 250") am proximalen Konusende (210, 210', 210") und/oder am distalen Konusende (220, 220', 220") durch Sägen, Schneiden oder Abbrechen entlang der radial umlaufenden Nut (400, 400', 400") oder Nuten (400, 400', 400").

10. Verfahren nach Anspruch 9 zur Adaption einer Augmentationsvorrichtung (100, 100', 100") nach Anspruch 7, umfassend einen Schritt eines Abtrennens eines Konusteilstücks (270, 270") aus dem Konus (200, 200', 200") durch Sägen, Schneiden oder Abbrechen entlang zweier der axial verlaufenden Axialnuten (430, 430").

## Claims

1. An augmentation device (100, 100', 100") comprising
an annular cone (200, 200', 200"), which surrounds a channel (300, 300') extending through the cone (200, 200', 200") from a proximal cone end (210, 210', 210") to a distal cone end (220, 220', 220") of the cone (200, 200', 200"),
and which consists of a biocompatible polymer by at least 50 volume percent relative to the total volume of the cone (200, 200', 200"),
**characterized in that**
the cone (200, 200', 200") is divided into annular cone segments (250, 250', 250") by at least three radially circumferential grooves (400, 400', 400") in a cone lateral surface (230, 230', 230") opposite the channel (300, 300'),
the grooves (400, 400', 400") having a groove depth (410) of at least 1 mm and a groove width (420) of at least 1 mm and forming sawing guides to adapt a cone size of the cone (200, 200', 200") by separating one or more cone segments (250, 250', 250").

2. The augmentation device (100, 100', 100") according to claim 1, **wherein** the cone lateral surface (230, 230', 230") is roughened or porous at least in portions.

3. The augmentation device (100') according to either claim 1 or claim 2, **wherein** the cone lateral surface (230') is made from tantalum, a tantalum alloy, titanium, a titanium alloy or from stainless steel by at least 70 area percent relative to the total cone lateral surface (230').

4. The augmentation device (100, 100', 100") according to any of the preceding claims,
**wherein** the biocompatible polymer is a PMMA bone cement.

5. The augmentation device (100, 100', 100") according to claim 4, **wherein** the PMMA bone cement contains at least one antibiotic.

6. The augmentation device (100, 100', 100") according to any of the preceding claims,
**wherein** the cone (200, 200', 200") has radially circumferential inner grooves in a cone inner surface (240, 240') facing the channel (300, 300'), which inner grooves are arranged opposite the grooves (400, 400', 400").

7. The augmentation device (100, 100', 100") according to any of the preceding claims, **wherein** the cone lateral surface (230, 230', 230") has at least two axially extending axial grooves (430, 430") having an axial groove depth of at least 1 mm and an axial groove width of at least 1 mm, which are each connected to at least one of the grooves (400, 400', 400").

8. The augmentation device (100) according to any of the preceding claims, **wherein** the cone segments (250") are offset from one another in a stepped manner.

9. A method for adapting a cone size of an augmentation device (100, 100', 100") according to any of claims 1 to 8, comprising a step of separating one or more cone segments (250, 250', 250") at the proximal cone end (210, 210', 210") and/or at the distal cone end (220, 220', 220") by sawing, cutting or breaking along the radially circumferential groove (400, 400', 400") or grooves (400, 400', 400").

10. The method according to claim 9 for adapting an augmentation device (100, 100', 100") according to claim 7, comprising a step of separating a cone part (270, 270") from the cone (200, 200', 200") by sawing, cutting or breaking along two of the axially extending axial grooves (430, 430").

## Revendications

1. Dispositif d'augmentation (100, 100', 100") comprenant
un cône (200, 200', 200") annulaire qui entoure un canal (300, 300'), lequel canal s'étend à travers le cône (200, 200', 200") à partir d'une extrémité de cône proximale (210, 210', 210") jusqu'à une extrémité de cône distale (220, 220', 220") du cône (200, 200', 200"),
et lequel cône est constitué à raison d'au moins 50 pour cent en volume, par rapport au volume total du cône (200, 200', 200"), d'un polymère biocompatible,
**caractérisé en ce que**
le cône (200, 200', 200") est divisé en segments de cône (250, 250', 250") annulaires par au moins trois rainures (400, 400', 400") radialement périphériques dans une surface d'enveloppe de cône (230, 230', 230") opposée au canal (300, 300'),
les rainures (400, 400', 400") présentant une profondeur de rainure (410) d'au moins 1 mm et une largeur de rainure (420) d'au moins 1 mm et formant des guide-lames afin d'adapter une dimension de cône du cône (200, 200', 200") par séparation d'un ou de plusieurs segments de cône (250, 250', 250").

2. Dispositif d'augmentation (100, 100', 100") selon la revendication 1, **dans lequel** la surface d'enveloppe de cône (230, 230', 230") est rendue rugueuse ou poreuse au moins dans certaines zones.

3. Dispositif d'augmentation (100') selon la revendication 1 ou 2, **dans lequel** la surface d'enveloppe de cône (230') est formée, au moins à raison de 70 pour cent de la surface, par rapport à la totalité de la surface d'enveloppe de cône (230'), de tantale, d'un alliage de tantale, de titane, d'un alliage de titane ou d'acier inoxydable.

4. Dispositif d'augmentation (100, 100', 100") selon l'une des revendications précédentes, **dans lequel** le polymère biocompatible est un ciment osseux à base de PMMA.

5. Dispositif d'augmentation (100, 100', 100") selon la revendication 4, **dans lequel** le ciment osseux à base de PMMA contient au moins un antibiotique.

6. Dispositif d'augmentation (100, 100', 100") selon l'une des revendications précédentes, **dans lequel** le cône (200, 200', 200") présente, dans une surface interne de cône (240, 240') orientée vers le canal (300, 300'), des rainures internes radialement périphériques qui sont disposées à l'opposé des rainures (400, 400', 400").

7. Dispositif d'augmentation (100, 100', 100") selon l'une des revendications précédentes, **dans lequel** la surface d'enveloppe de cône (230, 230', 230") présente au moins deux rainures axiales (430, 430") s'étendant axialement et comportant une profondeur de rainure axiale d'au moins 1 mm et une largeur de rainure axiale d'au moins 1 mm, lesquelles rainures axiales sont reliées respectivement au moins à l'une des rainures (400, 400', 400").

8. Dispositif d'augmentation (100) selon l'une des revendications précédentes, **dans lequel** les segments de cône (250") sont écartés de façon étagée les uns des autres.

9. Procédé pour l'adaptation d'une dimension de cône d'un dispositif d'augmentation (100, 100', 100") selon l'une des revendications 1 à 8, comprenant une étape de séparation d'un ou de plusieurs segments de cône (250, 250', 250") au niveau de l'extrémité de cône proximale (210, 210', 210") et/ou de l'extrémité de cône distale (220, 220', 220") par sciage, découpe ou rupture le long de la rainure (400, 400', 400") ou des rainures (400, 400', 400") radialement périphériques.

10. Procédé selon la revendication 9 pour l'adaptation d'un dispositif d'augmentation (100, 100', 100") selon la revendication 7, comprenant une étape de séparation d'un morceau partiel de cône (270, 270") du cône (200, 200', 200") par sciage, découpe ou rupture le long de deux rainures axiales parmi les rainures axiales (430, 430") s'étendant axialement.
